# EUROPEAN PATENT APPLICATION

(11) **EP 0 858 742 A2**
(43) Date of publication of application: **19.08.1998**
(21) Application number: 98301164.4
(22) Date of filing: 17.02.1998
(51) Int. Cl.: A23L 1/212, A23L 1/015, A23L 1/302

(54) **Nitrate-free powdered fruits or vegetables and manufacturing process therefor**

(30) Priority: 17.02.1997 JP 48383/97
(71) Applicant: M-P-G. CO., LTD., Tokyo (JP)
(72) Inventor: Sugisaki, Takashi, Kawaguchi-shi, Saitama-ken (JP)
(74) Representative: Matthews, Derek Peter

(57) **Abstract**

Vegetables and/or Fruit or macerated vegetables and/or fruit are washed with water which has passed through ion-exchange resins capable of removing NO₃₋ and NO₂₋ ions to remove nitrate nitrogen compounds which are harmful to the human body and then dried by freeze-drying.

## Description

The present invention relates to powdered vegetables and powdered fruits and a manufacturing process therefor, wherein said powdered vegetables or powdered fruit are free from nitrate nitrogen compounds harmful to the human body and are added to or mixed with other components helpful in maintaining health by, for example, preventing senescence and reinforcing blood vessels.

Recently, water pollution with nitrate nitrogen compounds has become a serious social concern together with ground water contamination with organochlorine compounds such as trichloroethylene. Nitrate nitrogen compounds which are contaminants of said ground water and also derive from nitrogen fertilizers and pesticides and also exist as nitrates and nitrites in organisms.

Thus, organic nitrogen contained in debris will penetrate the soil, decompose into ammonia nitrogen therein and subsequently be oxidized to nitrate via nitrite.

Therefore, nitrate nitrogen compounds are widely distributed in many plants, particularly in vegetables and fruit since nitrate nitrogen compounds are found in ground water. Although they mainly exist as nitrates in ground water, they are easily translocated together with ground water, or nitrate nitrogen derived from fertilizers or pesticides, taken up by plants and incorporated into organisms.

The toxicity of these nitrates to humans is believed to be due to reduction of nitrate by microorganisms in the body to nitrites. It is well known that said nitrites not only oxidize haemoglobin (which has an important role as an oxygen transporter in blood) to methaemoglobin (which lacks the oxygen transport ability, resulting in oxygen deficiency in various organs), but also causes adverse effect such as senescence of blood vessels. Further, it is known that nitrites react in the stomach with secondary amines which are non-carcinogens, to form nitrosoamines which are carcinogens.

With ordinary methods of obtaining powdered vegetables or powdered fruit for use in making vegetable and/or fruit juices or as food supplements, several methods exist; a method for freeze-drying fresh fruit or vegetables followed by pulverization using a mill, a method for drying fresh fruit or vegetables using a dryer followed by pulverization using a mill, another method of squeezing fresh fruit or vegetables avoiding decomposition of components, freeze-drying the resulting liquid followed by further pulverization using a mill. There are however no methods which deal with nitrate nitrogen compounds harmful to the human body, contained in or deposited on vegetables.

The object of the present invention is to provide powdered vegetables and/or powdered fruit and a manufacturing process therefor, wherein said powdered vegetables and/or fruit are free from nitrate nitrogen compounds which are harmful to the human body and contained in or deposited on fruit and vegetables, and which may be added to or mixed with other components effective in maintaining health.

In order to obtain such nitrate nitrogen free powdered fruit or vegetables, in the manufacturing process according to the present invention, fruit and/or vegetables, preferably macerated fruit or vegetables, are washed with water which has passed through an ion-exchange resin capable of removing NO³⁻ and NO²⁻ ions to remove nitrate nitrogen and subsequently freeze-dried and pulverized. The resultant powdered fruit or vegetables may be supplemented with components effective for maintaining a healthy life such as blood vessel reinforcers and various vitamins.

Further, powdered fruit and/or vegetables according to the present invention can usually be packed into an air-tight container, a sealable plastic bag for example, containing a volume sufficient to make one cup or glass, or into other types of containers and be used as a beverage after dissolving in water when necessary, or used as a supplement for various foods, for example infant food and hospital diets. They are also effective for solving deviant food habits, particular a dislike of fruit or vegetables.

Vegetables and fruit which may be used in the present invention include celery, komatsuna (Brassica campestris, rapifera group), chingensai (B. campestris, chinensis group), Garland chrysanthemum, parsley, lettuce, spinach, asparagus, carrot, Welsh onion, Chinese cabbage, cabbage, Japanese hornwort, Japanese mugwort, tomato and other vegetables and fruits such as apples.

The ion-exchange resins used to remove nitrate nitrogen compounds are anion-exchange resins capable of removing NO³⁻ and NO²⁻ ions, for example a strong basic ion-exchange resin (Cl-ion form).

Methods and results of nitrate nitrogen compound removal tests are presented below. The invention is not however limited to these methods.

First, the method and results of nitrate nitrogen compound removal test (1) used to remove nitrate nitrogen deposition on the surface of vegetables.

Nitrate Nitrogen Compound Removal Test (1)
(a) One hundred grams each of seven different vegetables (celery, komatsuna, chingensai, Garland chrysanthemum, parsley, lettuce and spinach) were weighed and equally divided into two portions.
(b) Seven containers filled with 6 liters of tap water treated with said ion-exchange resin (hereinafter referred to as "purified water") and seven containers filled with 6 liters of untreated tap water (hereinafter referred to as "tap water") were prepared.
(c) Each of said fresh vegetable samples was thoroughly washed by hand in each container for 3 minutes.
(d) After removal of the fresh vegetable sample from the container, the amount (mg/liter) of nitrate nitrogen in the washing water in the container was determined using pack-test equipment for water quality test (Kyouritsu Rika K.K.).
(e) Procedures (a) to (d) as above were repeated until the amount of nitrate nitrogen compound in said washing becomes 0.

Results obtained from the nitrate nitrogen compound removal test (1) are presented in Table 1 attached.

As is obvious from Table 1, when washing with purified water was repeated, washing efficiency increased every time and the amount of nitrate nitrogen in said washing water reduced to zero over 3 to 10 washes, depending on the vegetable used. Therefore, it was confirmed that nitrate nitrogen compounds deposited on fresh vegetables were completely removed. This was true for dried vegetable separately tested.

On the other hand, where washing was done with tap water, although it was expected that a small amount of nitrate nitrogen compound might be released into the washing at least for the first wash, a measurable amount of nitrate nitrogen compound was not detected, probably because the level was too low to detect by the determination method used. Even though said washing was repeated further, the presence of nitrate nitrogen compounds in the washing water could not be confirmed. Therefore, it was considered that nitrate nitrogen compounds were not removed from the fresh vegetables.

The method and results of nitrate nitrogen compound removal test (2) used to remove nitrate nitrogen deposited on the surface of and contained in vegetables is explained below.

Nitrate Nitrogen Compound Removal Test (2)
(a) One hundred grams each of 14 different vegetables or fruits (asparagus, tomato, carrot, apple, Welsh onion, celery, komatsuna, chingensai, Garland chrysanthemum, parsley, lettuce, spinach, Chinese cabbage and Japanese hornwort) were weighed out.
(b) Each of said fruit or vegetable samples was cut into a square pieces of about 1 x 1 cm and prepared in an almost crushed state in a mortar, care being taken to avoid decomposition of nutrients.
(c) 0.2 ml of the resulting extract of each vegetable or fruit sample was diluted with said purified water to 100 times. The concentration of nitrate nitrogen compounds in this diluted extract was determined spectroscopically using a simplified reflectance spectrometer RQ Flex (Kanto Chemical Co., Ltd.); the test paper was dipped into the diluted extract for 2 seconds, excess water on the test paper was removed and the paper was inserted into the equipment.
(d) Extracted residue (mainly fiber components) remaining after the extract was separated according to this method was diluted again with purified water to 100 times and the concentration of nitrate nitrogen compounds determined using said RQ Flex in a similar way to the above, to confirm the amount of nitrate nitrogen compounds remaining in the extracted residue of each vegetable or fruit sample.

Results of said nitrate nitrogen compound removal test (2) are presented in Table 2 attached.

As is obvious from Table 2, nitrate nitrogen compounds could be detected from each of said fresh fruit or vegetable extracts; 460 mg/liter from lettuce extract and a relatively high level of 1311 mg/liter from Japanese hornwort. It was however confirmed that the level of nitrate nitrogen compounds in the purified water extracted residue of each vegetable or fruit sample, was only of trace levels and below the detection limit of 0.3 mg/liter.

As is mentioned above, the manufacturing process for producing powdered vegetables or fruit including the steps to removing nitrate nitrogen compounds on the surface of fruit or vegetables according to said nitrate nitrogen compound removal test (1), followed by drying using a freeze-dryer as is commercially available, then adding various components effective for maintaining a healthy life such as blood vessel reinforcers and various vitamins, can provide powdered vegetables and/or fruit containing a significantly lower level of nitrate nitrogen compound than other ordinary methods.

However, nitrate nitrogen compounds can also exist in vegetable or fruit tissue. Preferably therefore, vegetables and fruit are cut as finely as possible to prepare macerated fruit and vegetables, prepared to an almost crushed state, washed with purified water according to said nitrate nitrogen compound removal test (2), dried using a freeze-dryer and then pulverized using, for example, a mill, and then constituted by adding other various components are effective for maintaining a healthy life such as blood vessel reinforcers and various vitamins.

As blood vessel reinforcers may be used for example, rutin (synonym: vitamin P) which reinforces the connective tissue of capillary blood vessels and can be used in order to prevent senescence and for reinforcement of blood vessels, chondrithion sulphuric acid (synonym: chondroitin-protein complex) which activates arterial wall cells and can be used in arterial sclerosis, particularly in coronary artery sclerosis, also proanthocyazine which is active in preventing hyperoxidation of lipids and in eliminating active oxygen can be used. As nutritional supplements, vitamins such as vitamin A, B₁, B₂, B₆, B₁₂, E, C, niacin and folic acid can be added. Furthermore, zinc, iron, calcium and other nutrients essential for healthy maintenance of the human body can be added.

As aforementioned, powdered vegetables and powdered fruit according to the present invention are safe to the human body and exhibit the health maintaining goodness existing in fruit and vegetables when used not only in making up vegetable and fruit juices but also as infant foods and hospital food after being dissolved in water, because said fruit or vegetable juice is constituted with fruit or vegetables after the nitrate nitrogen compounds harmful to the human body are eliminated.

Further, because components effective for a healthy life such as blood vessel reinforcers and various vitamins can be added, powdered vegetables or powdered fruit according to the present invention provides various advantage effects such as prevention of blood vessel senescence, by for example elimination of active oxygen, improved blood circulation, prevention of senescence of blood vessels due to increase of hyperoxidized lipid, enhancement of capillary blood vessels, decrease of serum cholesterol and the like. Powdered vegetables or powdered fruit according to the present invention also provides reinforcing effect on blood vessels and tissue cells.

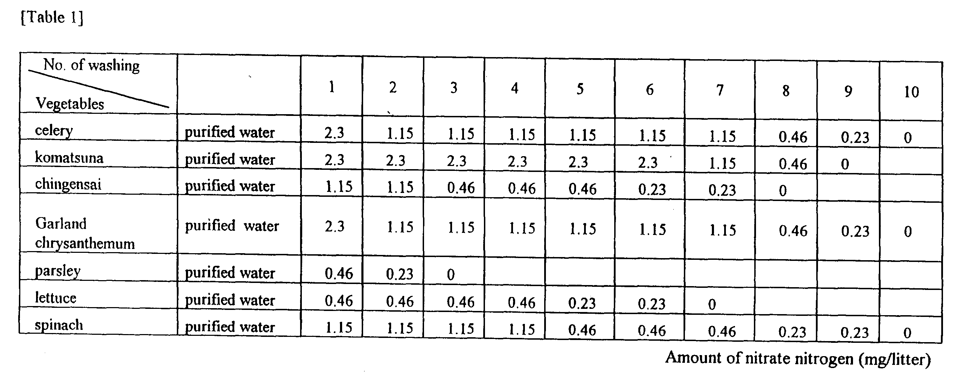

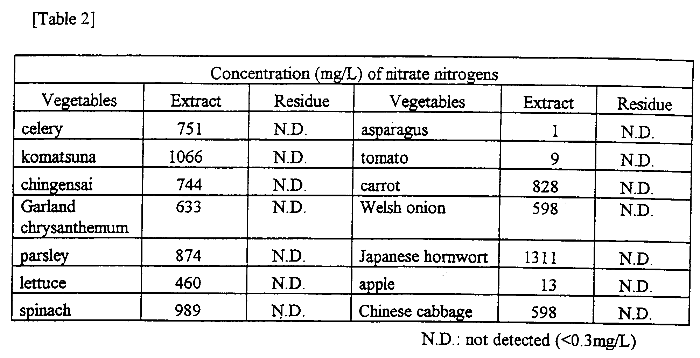

## Claims

1. Powdered fruit and/or vegetables, characterised in that the powdered vegetables or fruit are free from nitrate nitrogen compounds, and wherein said powdered vegetables and/or fruit are optionally mixed with or added to blood vessel reinforcers and/or various vitamins.

2. A manufacturing process for powdered vegetables and/or powdered fruit, characterised in that the vegetables and/or fruit are washed with water which has passed through ion-exchange resins capable of removing NO³⁻ and NO²⁻ ions, said washed vegetables and/or fruit are dried by freeze-drying and pulverized using, for example, a mill and are optionally added to or mixed with blood vessel reinforcers and/or various vitamins.

3. A manufacturing process for powdered vegetables and/or powedered fruit as described in claim 2, characterised in that said fruit and/or vegetables are macerated.

4. Fruit and/or vegetable juice prepared by reconstitution of a powdered fruit and/or vegetable according to claim 1.
